# EUROPEAN PATENT SPECIFICATION

(11) **EP 3 423 011 B1**
(45) Date of publication and mention of the grant of the patent: **08.07.2020**
(21) Application number: 17702713.3
(22) Date of filing: 16.01.2017
(51) Int. Cl.: A61F 13/00, A61F 13/02

(54) **DRAPE FOR USE WITH MEDICAL THERAPY SYSTEMS**
ABDECKTUCH ZUR VERWENDUNG IN MEDIZINISCHEN THERAPIESYSTEMEN
CHAMP OPÉRATOIRE POUR UTILISATION AVEC DES SYSTÈMES DE THÉRAPIE MÉDICALE

(30) Priority: 01.03.2016 US 201662301900 P
(43) Date of publication of application: 09.01.2019
(60) Divisional application: 20154188.5
(73) Proprietor: KCI Licensing, Inc., San Antonio, TX 78265 (US)
(72) Inventor: LOCKE, Christopher, Brian, Bournemouth BH9 3SD (GB); ROBINSON, Timothy, Mark, Shillingstone DT11 0TG (GB)
(74) Representative: Simmons & Simmons
(86) International application number: PCT/US2017/013642
(87) International publication number: WO 2017/151226

(56) References cited:
- EP-A1- 2 436 348
- WO-A1-2014/202935
- GB-A- 2 312 214
- US-A1- 2009 216 170
- US-A1- 2015 119 834

## Description

### TECHNICAL FIELD

The invention set forth in the appended claims relates generally to tissue treatment systems and more particularly, but without limitation, to a drape having a first bond strength, a second bond strength, and a third bond strength.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed. WO 2014/202935 A1 concerns a medical skin covering comprising a layer of switchable adhesive. US 2015/119834 A1 concerns a dressing for treating a tissue site including a tissue interface and sealing member. US 2009/216170 A1 concerns a system and method for facilitating removal of a drape from a tissue site. EP 2436348 A1 concerns a wound dressing for managing wound fluids including a porous contact layer.

While the clinical benefits of negative-pressure therapy and/or instillation therapy are widely known, improvements to therapy systems, components, and processes may benefit healthcare providers and patients.

### BRIEF SUMMARY

New and useful systems and apparatuses for covering a tissue site are set forth in the appended claims. Illustrative embodiments are also provided to enable a person skilled in the art to make and use the claimed subject matter.

For example, in some embodiments, a drape for medical applications is described according to claim 1.

More generally, a system for providing negative-pressure therapy is described according to claim 20.

The drape and system of the invention can be used in a method for treating a tissue site. A tissue interface can be positioned over the tissue site, and a sealing member can be position over the tissue interface. The sealing member can include a first layer having a first bond strength prior to application of the sealing member, a second bond strength in response to a force applied to the sealing member, and a third bond strength following exposure of the first layer to electromagnetic radiation in a visible light spectrum. The sealing member can also include a second layer coupled to the first layer and a third layer releasably coupled to the second layer opposite of the first layer. The third layer can be configured to block passage of at least a portion of the electromagnetic radiation in the visible light spectrum. A force can be applied to the sealing member to transition the first layer from the first bond strength to the second bond strength, sealing the sealing member to an attachment surface surrounding the tissue site. A negative-pressure source can be fluidly coupled to the tissue interface and negative-pressure therapy can be conducted. The third layer can be removed from the sealing member, and in response to ambient light, the first layer can transition from the second bond strength to the third bond strength. The sealing member can then be removed.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure therapy or instillation therapy in accordance with this specification;
Figure 2 is a sectional perspective view of a portion of a cover illustrating additional details that may be associated with an example embodiment of the therapy system of Figure 1;
Figure 3 is a sectional view of the cover of Figure 2, illustrating additional details that may be associated with the use of the cover;
Figure 4 is a sectional view of the cover of Figure 2 having a barrier layer removed;
Figure 5 is a sectional view of another cover that may be used with the therapy system of Figure 1;
Figure 6 is a sectional view of another cover that may be used with some embodiments of the therapy system of Figure 1; and
Figure 7 is a sectional view of another cover that may be used with some embodiments of the therapy system of Figure 1.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

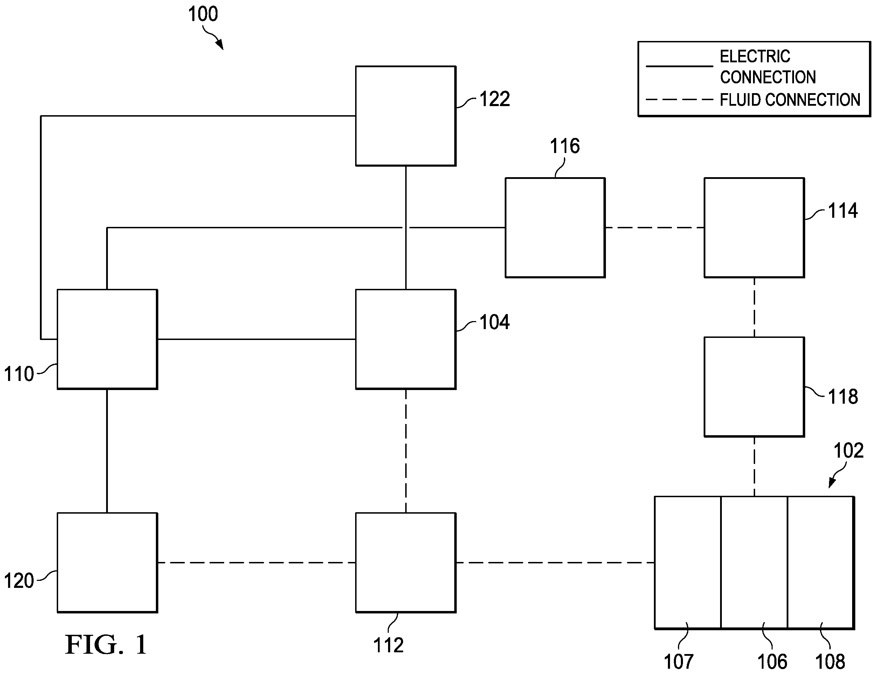

Figure 1 is a simplified functional block diagram of an example embodiment of a therapy system 100 that can provide negative-pressure therapy with instillation of topical treatment solutions in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the negative-pressure source 104.

In some embodiments, a dressing interface 107 may facilitate coupling the negative-pressure source 104 to the dressing 102. For example, such a dressing interface may be a T.R.A.C.® Pad or Sensa T.R.A.C.® Pad available from Kinetic Concepts, Inc. ("KCI") of San Antonio, Texas. The therapy system 100 may optionally include a fluid container, such as a container 112, coupled to the dressing 102 and to the negative-pressure source 104.

The therapy system 100 may also include a source of instillation solution. For example, a solution source 114 may be fluidly coupled to the dressing 102, as illustrated in the example embodiment of Figure 1. The solution source 114 may be fluidly coupled to a positive-pressure source such as the positive-pressure source 116 in some embodiments, or may be fluidly coupled to the negative-pressure source 104. A regulator, such as an instillation regulator 118, may also be fluidly coupled to the solution source 114 and the dressing 102. In some embodiments, the instillation regulator 118 may also be fluidly coupled to the negative-pressure source 104 through the dressing 102.

Additionally, the therapy system 100 may include sensors to measure operating parameters and provide feedback signals to the controller 110 indicative of the operating parameters. As illustrated in Figure 1, for example, the therapy system 100 may include a pressure sensor 120, an electric sensor 122, or both, coupled to the controller 110. The pressure sensor 120 may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112.

In general, components of the therapy system 100 may be coupled directly or indirectly. For example, the negative-pressure source 104 may be directly coupled to the controller 110 and may be indirectly coupled to the dressing 102 through the container 112.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, the negative-pressure source 104 may be combined with the controller 110 and other components into a therapy unit. A negative-pressure supply may also have one or more supply ports configured to facilitate coupling and de-coupling the negative-pressure supply to one or more distribution components.

A negative pressure supply may include or may be communicatively coupled to a controller, such as the controller 110. A controller may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller 110 is also preferably configured to receive one or more input signals, such as a feedback signal, and programmed to modify one or more operating parameters based on the input signals.

Sensors, such as the pressure sensor 120 or the electric sensor 122, are generally known in the art as apparatuses operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the negative-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

A positive-pressure source, such as the positive-pressure source 116, may be a reservoir of air at a positive pressure, or may be a manual or electrically-powered device that can increase the pressure in a sealed volume. A positive-pressure source may be housed within or used in conjunction with other components, such as sensors, processing units, alarm indicators, memory, databases, software, display devices, or user interfaces that further facilitate therapy. For example, the positive-pressure source 116 may be combined with the controller 110, the solution source 114, the instillation regulator 118, and other components into a therapy unit. A positive-pressure source may also have one or more supply ports configured to facilitate coupling and de-coupling the positive-pressure source to one or more distribution components.

An instillation regulator, such as the instillation regulator 118, may be a mechanical or electromechanical device configured to control a fluid flow. An instillation regulator can include valves, microprocessors, or other components configured to control a positive pressure of fluid into a sealed therapeutic environment.

The tissue interface 108 can be adapted to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of foam may vary according to needs of a prescribed therapy. For example, in some embodiments, the tissue interface 108 may be foam having pore sizes in a range of about 400 to about 600 microns. The tensile strength of the tissue interface 108 may also vary according to needs of a prescribed therapy. For example, the tensile strength of foam may be increased for instillation of topical treatment solutions. In one non-limiting example, the tissue interface 108 may be an open-cell, reticulated polyurethane foam such as GranuFoam® dressing or VeraFlo® foam, both available from KCI of San Antonio, Texas.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of hydrophilic foam is a polyvinyl alcohol, open-cell foam such as V.A.C. WhiteFoam® dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The tissue interface 108 may further promote granulation at a tissue site when pressure within the sealed therapeutic environment is reduced. For example, any or all of the surfaces of the tissue interface 108 may have an uneven, coarse, or jagged profile that can induce microstrains and stresses at a tissue site if negative pressure is applied through the tissue interface 108.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

The solution source 114 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 can include, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least about 300 g/m² per twenty-four hours. In some example embodiments, the cover 106 may include a polymer drape, such as a polyurethane film, that is permeable to water vapor but impermeable to liquid. Such drapes typically have a thickness in the range of about 25 microns to about 50 microns. For permeable materials, the permeability generally should be low enough that a desired negative pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, some or all of the cover 106 may be coated with an acrylic adhesive having a coating weight between about 25 grams per square meter (gsm) and about 70 gsm. Thicker adhesives, or combinations of adhesives, may be applied in some embodiments to improve the seal and reduce leaks. For example, in some embodiments, a film layer may be coated with an acrylic adhesive having a coating weight between about 100 gsm and about 150 gsm. Other example embodiments of an attachment device may include a double-sided tape, paste, hydrocolloid, hydrogel, silicone gel, or organogel.

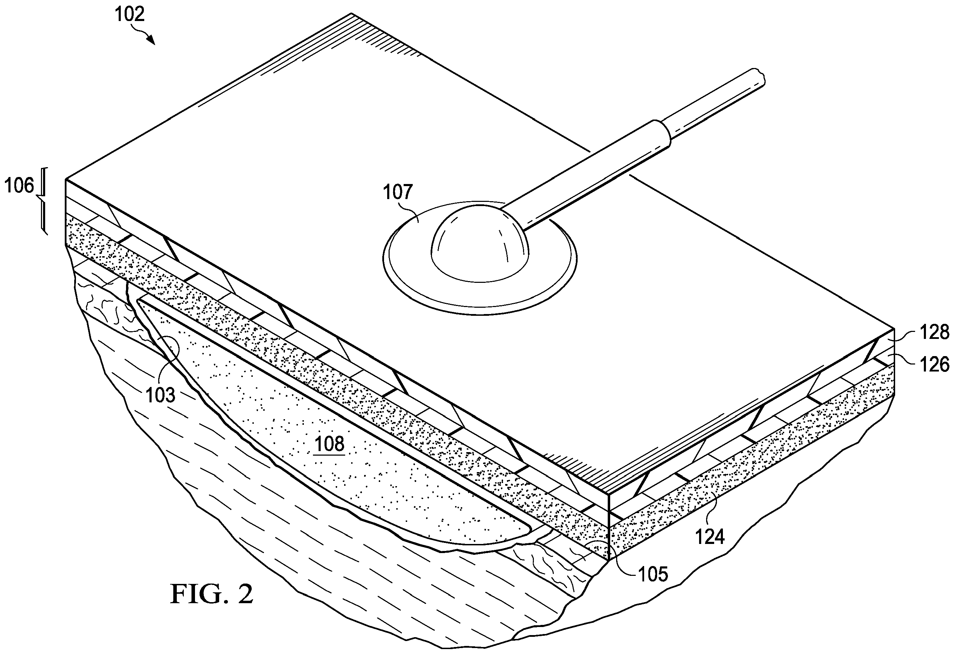

Figure 2 is a sectional perspective view of a portion of the dressing 102, illustrating additional details that may be associated with some embodiments. The tissue interface 108 may be placed within, over, on, or otherwise proximate to a tissue site 103. The cover 106 may be placed over the tissue interface 108 and sealed to an attachment surface 105 near the tissue site 103. For example, the cover 106 may be sealed to undamaged epidermis peripheral to a tissue site. Thus, the dressing 102 can provide a sealed therapeutic environment proximate to the tissue site 103, substantially isolated from the external environment. The negative-pressure source 104 can be fluidly coupled to the dressing 102 through the dressing interface 107 to reduce the pressure in the sealed therapeutic environment. Negative pressure applied across the tissue site 103 through the tissue interface 108 in the sealed therapeutic environment can induce macrostrain and microstrain in the tissue site 103, as well as remove exudates and other fluids from the tissue site 103, which can be collected in the container 112.

To ensure that a cover can maintain a sealed therapeutic environment over a tissue site, the attachment device may comprise an adhesive having high tackiness, high bond strength, or both high tackiness and high bond strength. A high bond strength adhesive is an adhesive that may readily bond to tissue and provide a seal between the cover and the tissue site. Tack describes a property of an adhesive that relates a particular bond strength of an adhesive on a substrate to a time period necessary to achieve the particular bond strength.

Generally, covers can be difficult to handle when a user attempts to place a cover over a tissue site. It is often necessary for a cover to be sized and positioned over an area that is larger than the area of a tissue site to ensure a good seal for the sealed therapeutic environment. Because of the type and location of a tissue site and the surface area of a cover, the cover may become inadvertently attached to itself. For example, a portion of a cover may become folded over so that an adhesive of the cover contacts and bonds to itself. If the adhesive bonds to itself, separating the bonded surfaces of the adhesive can be difficult to do without damaging the cover. In some cases, pulling the bonded surfaces apart may form irreparable tears or create other discontinuities in the cover. As a result, the cover must be discarded because the cover might not provide and maintain a sealed therapeutic environment over a tissue site as desired.

Even if a cover does not become attached to itself during positioning of a cover over a tissue site, the cover may inadvertently attach to an undesired attachment surface or simply not be properly positioned. If the cover becomes attached to an undesired attachment surface or is not properly positioned to form a sealed therapeutic environment over the tissue site, a user may find it difficult to remove and reposition the cover without damaging the cover.

For tissue sites that may be located near or cover curved portions of a patient, a user may have a difficult time creating a seal between a cover and an attachment surface. To address such difficulties, users may opt to cover the edges of a cover with portions of another cover or with a medically approved tape. However, some high bond adhesives used for covers do not adhere well to the film used to form the cover, particularly if the material of the film comprises polyurethane.

A cover having a high bond strength adhesive can also be difficult to remove during or following therapy. Adhesives strong enough to adhere to tissue during negative-pressure therapy or instillation therapy can often cause pain and tissue damage to a patient during removal. As many negative-pressure therapy or instillation therapy regimes may require the removal and replacement of a cover during therapy, the cover may become worn or damaged and the patient may experience more pain than necessary.

Furthermore, many high bond strength adhesives lose tack if exposed to moisture, such as the sweat from a patient's body form at the attachment surface. As a result, a cover that initially held a seal may lose contact with the attachment surface as a patient perspires resulting in a leak. Similarly, some high bond strength adhesives used in covers may lose tack in the presence of bodily heat. Again, the cover that initially held a seal may lose contact with the attachment surface as a patient's body temperature heats up the adhesive resulting in a leak.

The cover 106 of the therapy system 100 addresses these issues and others by providing a cover having an adhesive with a first bond strength that holds the cover over the tissue site 103 while the cover 106 is being positioned and can be removed without damaging the attachment surface 105 or other tissues. The adhesive of the cover 106 may also have a second bond strength that can affix the cover 106 to the attachment surface 105 over the tissue site 103 to form a sealed therapeutic environment and prevent leakage of negative pressure and other fluids from the sealed therapeutic environment. The adhesive of the cover 106 may also have a third bond strength that permits the cover 106 to be removed from the tissue site 103 while minimizing instances of pain or other trauma to a patient. In an exemplary embodiment, the cover 106 may include a first layer or attachment layer, such as an adhesive layer 124, a second layer or backing layer, such as a film layer 126, and a third layer or filter layer, such as a barrier layer 128.

The adhesive layer 124 may be an acrylic adhesive, a polyurethane adhesive, a hydrocolloid, a hydrogel, a silicone gel, or an organogel. The adhesive layer 124 may also be pressure-sensitive. A pressure-sensitive adhesive is an adhesive having a bond strength that increases if pressure is applied to the adhesive. The increase in bond strength of a pressure-sensitive adhesive may be driven by crosslinking. Crosslinking occurs by chemically joining two or more molecules by a covalent bond linking one polymer chain to another polymer chain. The bond strength of the adhesive increases as the crosslinking increases until the adhesive reaches a maximum bond strength. Any further crosslinking may deteriorate the bond strength of the adhesive. A curve plotting adhesive bond strength vs. an amount of crosslinking, where the adhesive bond strength is on the y-axis and the amount of crosslinking is on the x-axis, illustrates that the maximum bond strength of an adhesive will generally occur at a midpoint of the curve along the x-axis as is well understood by one skilled in the art.

If a force is applied to a pressure-sensitive adhesive, the force may increase the crosslinking, thereby increasing the bond strength of the adhesive. In some example embodiments, the pressure-sensitive adhesive also may be a flowable adhesive or a gap-filling adhesive, wherein the adhesive is sufficiently viscous to remain on a substrate and also sufficiently non-viscous to fill any gaps or leaks that may form between the cover 106 and the attachment surface 105. If a force applied directly or indirectly to a pressure-sensitive adhesive or flowable adhesive, the force applied can reduce the viscosity of the adhesive to better fill crevices and gaps between the attachment surface 105 and the cover 106. In some embodiments, crosslinking can be controlled during application of the adhesive to enhance the flowable characteristic of the adhesive and increase the bond strength up to the maximum bond strength value. Crosslinking can be controlled by including crosslinking agents, which may be encapsulated, that are sensitive to an expected force applied to the adhesive.

In some example embodiments, the adhesive layer 124 may be an acrylic adhesive having a coating weight in a range between about 15 g/m² (gsm) and about 70 gsm. The adhesive layer 124 may have a thickness in a range between about 30 microns and about 70 microns. In other example embodiments, the adhesive layer 124 may have a thickness in a range between about 100 microns and about 150 microns. In some example embodiments, an increase in a thickness of the adhesive layer 124 may provide additional adhesive to flow into crevices between the cover 106 and the attachment surface 105. The additional adhesive can fill crevices to increase sealing of the sealed therapeutic environment and reduce leakage of negative pressure and other fluids through the interface between the cover 106 and the attachment surface 105.

The bond strength of adhesive materials may be measured in terms of a force necessary to remove the adhesive from a substrate. The force necessary to remove the adhesive from a substrate can also be referred to as a peel adhesion or resistance to being peeled. The American Society for Testing and Materials ("ASTM") standardized peel adhesion under ASTM D3330 as the force necessary to remove an adhesive from a stainless steel substrate at 23° C and 50% relative humidity. The tack or tackiness of an adhesive material may be measured by the amount of time it takes the adhesive to reach the desired bond strength. For example, a high tack means that the adhesive increases to the desired bond strength over a relatively short period of time. In one example embodiment, the adhesive layer 124 may have a first bond strength in a range between about 0.5N/25mm and about 1.5N/25mm. In some example embodiments, the adhesive layer 124 may have a low to medium tackiness wherein the adhesive layer 124 may achieve the first bond strength after a contact time of less than 60 seconds.

In some embodiments, the adhesive layer 124 may be a pressure-sensitive adhesive having a bond strength that increases if pressure is applied to the adhesive layer. Consequently, the bond strength of the adhesive layer 124 may transition from the first bond strength to a second bond strength when pressure or force is applied, directly or indirectly, to the adhesive layer 124. In some example embodiments, the second bond strength may be greater than the first bond strength. For example, the second bond strength may have a peel adhesion or resistance to being peeled in a range between about 6N/25mm and about 10N/25mm.

In some embodiments, tackifiers, low surface tension additives, adhesive softeners, or other crosslinking agents may be disposed within the adhesive layer 124. Tackifiers may increase the tackiness of the adhesive layer 124 if activated. Low surface tension additives may increase the flowability of the adhesive layer 124 if activated. In some embodiments, the tackifiers, low surface tension additives, or other crosslinking agents can be triggered by a pressure applied, directly or indirectly, to the exterior of the adhesive layer 124. Activation of tackifiers, low surface tension additives, or other crosslinking agents in the adhesive layer 124 can increase crosslinking, aiding the transition of the adhesive layer 124 from the first bond strength to the second bond strength.

In some embodiments, the tackifiers, low surface tension additives, or other crosslinking agents may be contained in microcapsules. The application of pressure or force, directly or indirectly, to the exterior of the adhesive layer 124 may rupture the microcapsules, causing the additives to be released to interact with the adhesive of the adhesive layer 124. In some embodiments, release from the microcapsules may cause the additives to react in a localized manner, creating high tack locations at the interface of the adhesive layer 124. In other embodiments, the bond strength of the adhesive layer 124 may be increased by chemical means resulting from the release of active agents into the adhesive layer 124.

The adhesive layer 124 may also be a switchable adhesive. A switchable adhesive is an adhesive that may have a high bond strength during use of the adhesive, but can be selected or switched to decrease in bond strength if the adhesive is no longer needed. One manner in which an adhesive can be switched is through the use of crosslinking. For example, once an adhesive has been crosslinked to its maximum bond strength, e.g., from the first bond strength to the second bond strength, the adhesive may be further crosslinked so that it becomes brittle to aid in the removal of the cover. In some instances, an adhesive may be further crosslinked by using a switching source. The switching source may be, for example, a liquid solution, an inert gas, or an electromagnetic radiation source. Alternatively, in other example embodiments, electromagnetic radiation such as, for example, visible light or ultraviolet light may be used to increase crosslinking of an adhesive beyond its maximum bond strength.

In some example embodiments, the adhesive layer 124 may include a photoinitiator or photosensitive agents. Photosensitive agents may be compounds disposed within the adhesive layer 124 that react to electromagnetic radiation of one or more wavelengths or a range of wavelengths. For example, the adhesive layer 124 may include a photosensitive agent configured to react to light in the violet, indigo, blue spectrum (blue spectrum). The blue spectrum may include electromagnetic radiation having wavelengths that coincide with violet, indigo, and blue wavelengths between about 380 nm and about 495 nm, and preferably about 470 nm. Upon exposure to the blue spectrum, the photosensitive agents may become active and react with the adhesive layer 124. The reaction between the photosensitive agents and the adhesive layer 124 advances crosslinking, thereby transitioning the bond strength of the adhesive layer 124 from the second bond strength to a third bond strength. In some embodiments, the third bond strength may be about 50% of the strength of the second bond strength.

Illustrative, but not limiting, examples of photosensitive agents may include camphorquinone such as Genocure ® CQ from Rahn AG and 5, 7-diiodo-3-butoxy-6-fluoroene, such as H-NU470 from Spectra Group Limited, Inc. The representative photosensitive agents provide through-cure in long wavelength electromagnetic radiation and are soluble in alcohol, ketones, acrylates, and methacrylates. The representative photosensitive agents may also have a molecular weight of 520 g/mol, a melting point of greater than 270 Celsius, a maximum wavelength absorbance of about 470 nm, and a molar extinction coefficient of 30,200. More generally, the photosensitive agents have panchromatic absorbance of electromagnetic radiation having wavelengths in the ultraviolet and visible light spectrums and, in particular, wavelengths in the 350 nm to 670 nm range. In some embodiments, the photosensitive agents can cure acrylates and epoxides. Suitable photosensitive agents can have high absorbency of electromagnetic radiation and may be used in low solution concentrations. For example, suitable concentrations of a photosensitive agent within an adhesive solution may be about 0.1-0.15% of the adhesive solution. Representative photosensitive agents may be capable of cure through greater than 1 inch of material and, in some embodiments, can be cured through ultraviolet, opaque, pigmented, or colored substrates, and can change colors after being cured.

Indoor illumination produces electromagnetic radiation that covers the visible light spectrum and generally includes a wavelength of 450 nm. Indoor illumination can include halogen lamps, incandescent lamps, metal halide lamps, sodium lamps, and fluorescent lamps. In some embodiments, a peak sensitivity of the photosensitive agents can be a wavelength of about 450 nm. Thus, indoor illumination within the 450 nm wavelength can activate the photosensitive agents, causing the adhesive layer 124 to crosslink and transition from the second bond strength to the third bond strength within at least ten minutes. In another exemplary embodiment, if the adhesive layer 124 is exposed to natural sunlight, crosslinking caused by the photosensitive agents can transition the adhesive layer 124 from the second bond strength to the third bond strength in less than ten minutes, for example, in a range between about three minutes and about six minutes.

In an illustrative, but not limiting, example, the adhesive layer 124 may comprise a switchable adhesive such as an Adhelight adhesive available from Lumina Adhesives AB located at Varbergsgatan 2A, 412 65 Göteborg, Sweden. The adhesive layer 124 comprising an Adhelight adhesive may be configured to switch in response to exposure to electromagnetic radiation. In some example embodiments, the adhesive layer 124 comprising an Adhelight adhesive may have a second bond strength of about 35 N/25 mm before exposure to electromagnetic radiation of a specific wavelength, and a third bond strength of about 4 N/25 mm after exposure to the electromagnetic radiation of the specific wavelength. In other example embodiments, the adhesive layer 124 comprising an Adhelight adhesive may have a second bond strength of about 2.5 N/25 mm and can be decreased to a third bond strength of about 0.5 N/25 mm after exposure to the electromagnetic radiation of a specific wavelength.

The film layer 126 may be a water vapor breathable film. For example, the film layer 126 may have a moisture vapor transmission rate (MVTR), tested using the inverted cup technique, of about 15,000 to 16,000 g/m2/24 hours. Using the upright cup technique, the film layer 126 may have a MVTR of about 3,000 to about 4,000 g/m²/24 hours. The tensile strength of the film layer 126 may range from about 800 g/25 mm to about 1200 g/25 mm and have an percentage elongation between about 400% and 440%. In some embodiments, the film layer 126 may be clear. In other embodiments, the film layer 126 may be translucent. The film layer 126 may be formed from polyurethane and have a thickness of about 15 microns. In some embodiments, the film layer 126 may be substantially liquid impermeable. In an illustrative, but not limiting, example, the film layer 126 may be Inspire® 2151 from Coveris Advanced Coatings. In some embodiments, the film layer 126 may include a carboxymethyl cellulose (CMC). A CMC is a cellulose derivative that may absorb liquid that may be proximate to the film layer 126 or, in the case of water vapor, may be permeating through the film layer 126. In some embodiments, sodium and potassium may readily interact with a CMC.

The barrier layer 128 may be a water vapor breathable film. In some embodiments, the barrier layer 128 may be formed from polyurethane, polyamide, polyether block amide (PEBA), ethylene-vinyl acetate (EVA), polyvinyl alcohol, or hydroxy or carboxy modified acrylics. The barrier layer 128 may have a high MVTR. For example, the barrier layer 128 may have an MVTR, tested using the inverted cup technique, of about 15,000 to 16,000 g/m2/24 hours. Using the upright cup technique, the barrier layer 128 may have a MVTR of about 3,000 to about 4,000 g/m²/24 hours. The tensile strength of the barrier layer 128 may range from about 800 g/25 mm to about 1,200 g/25 mm and have a percentage elongation between about 400% and 440%. In some embodiments, the barrier layer 128 may be transparent. In other embodiments, the barrier layer 128 may be translucent. The barrier layer 128 may be formed from polyurethane and have a thickness of about 15 microns. The barrier layer 128 may be liquid impermeable. In an illustrative, but not limiting, example, the barrier layer 128 may be Inspire® 2151 from Coveris Advanced Coatings.

In some embodiments, the barrier layer 128 may be colored, dyed, or tinted. A dye or coloring can be added during the manufacturing process of the barrier layer 128, causing the barrier layer 128 to transmit only light having a wavelength visible as the color of the dye or coloring. Other electromagnetic radiation is absorbed by the coloring or dye. The barrier layer 128 may remain substantially transparent following the dying process. In some embodiments, the barrier layer 128 may be tinted with a yellow dye or coloring. The yellow coloring may transmit electromagnetic radiation having a wavelength between about 570 nm and about 620 nm, filtering or blocking other electromagnetic radiation wavelengths. In other embodiments, the barrier layer 128 may be dyed, tinted, or colored so that the barrier layer 128 may transmit different wavelengths of the electromagnetic radiation spectrum.

The cover 106 may be constructed by providing the film layer 126 and coating a first side of the film layer 126 with an adhesive to form the adhesive layer 124. Additives, such as microcapsules, tackifiers, low surface tension additives, or photosensitive agents may be disposed within the adhesive prior to coating of the film layer 126. The adhesive layer 124 may be cured or dried, and the barrier layer 128 can be bonded to a second side of the film layer 126 opposite the adhesive layer 124. Prior to bonding of the barrier layer 128 to the film layer 126, the barrier layer 128 may be dyed or colored. In other embodiments, the adhesive layer 124 may be pattern printed, cast or otherwise formed on the film layer 126.

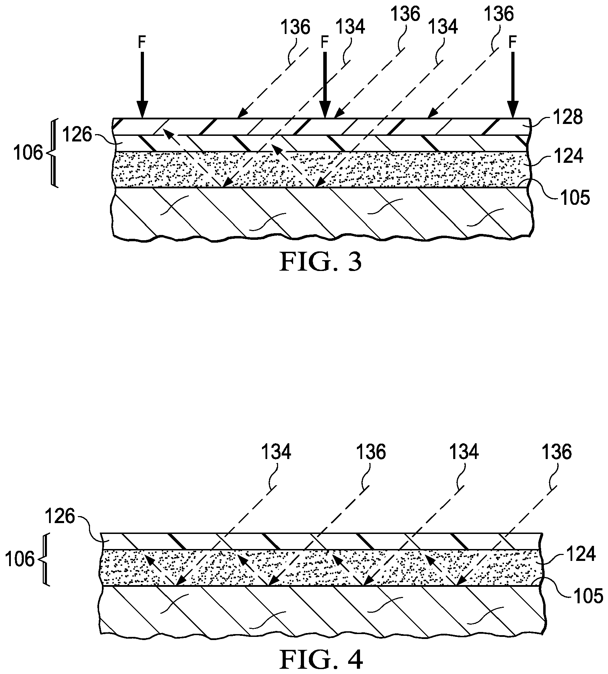

Figure 3 is a sectional view of a portion of the cover 106 positioned on the attachment surface 105 and illustrates additional details that may be associated with some embodiments. The cover 106 may be positioned over the tissue site so that a portion of the surface of the adhesive layer 124 opposite the film layer 126 contacts the attachment surface 105. In some embodiments, the first bond strength of the adhesive layer 124 may hold the cover 106 in place, yet permit the cover 106 to be removed from the attachment surface 105 without irritating or damaging the attachment surface 105 and/or causing pain to the patient. If the cover 106 is suitably positioned so that the adhesive layer 124 is positioned to provide a satisfactory seal, a force F may be applied to the barrier layer 128 to transition the adhesive layer 124 from the first bond strength to the second bond strength. For example, a user may apply pressure or force F with a hand or fingers directly to the barrier layer 128. In some embodiments, a user may apply the force F to the barrier layer 128 to selected areas of the cover 106. For example, a user may apply the force F to the cover 106 over the attachment surface 105. In other embodiments, a user may apply the force F over substantially all of the cover 106. For example, a user may apply the force to portions of the cover 106 over the attachment surface 105, the tissue site 103, and the tissue interface 108. The force F may cause crosslinking of the adhesive layer 124 that causes the adhesive layer 124 to transition from the first bond strength to the second bond strength. The force F may also increase the flow of the adhesive layer 124 into crevices and other locations of potential leaks between the cover 106 and the attachment surface 105. In some embodiments, the second bond strength of the adhesive layer 124 may be greater than the first bond strength to secure the cover 106 over the tissue site 103 and inhibit the inadvertent removal of the cover 106 from the attachment surface 105 during therapy.

In some embodiments, the barrier layer 128 may be selected to filter some electromagnetic radiation and, more specifically, some electromagnetic radiation within the visible light spectrum, from passing through the barrier layer 128. By filtering some electromagnetic radiation within the visible light spectrum, the barrier layer 128 can prevent the filtered electromagnetic radiation from prematurely activating the adhesive layer 124. As shown in Figure 3, the barrier layer 128 may be selected to absorb electromagnetic radiation having a wavelength between about 400 nm and about 475 nm, i.e., violet-blue light 136, and prevent the violet-blue light 136 from being transmitted through the barrier layer 128. By preventing the violet-blue light 136 from transmitting through the barrier layer 128 to the adhesive layer 124, the barrier layer 128 prevents activation of the photosensitive agents disposed in the adhesive layer 124. As a result, the adhesive layer 124 may maintain the second bond strength to ensure that the cover 106 is not inadvertently removed.

In other embodiments, the barrier layer 128 may be selected to transmit electromagnetic radiation and other wavelengths such as, for example, wavelengths between about 570 nm and about 750 nm and, more specifically, 570 nm and about 590 nm, i.e., visible light 134. Much of the visible light 134 would be transmitted through the barrier layer 128 and the adhesive layer 124 and then reflected by the attachment surface 105, the tissue site 103, and/or the tissue interface 108, and then back through adhesive layer 124 and the barrier layer 128 causing the barrier layer 128 to appear yellow. Even though the barrier layer 128 would appear to be yellow in color, the visible light 134 being reflected would still provide an image allowing a user to see through the cover 106 and continue monitoring the tissue site during the application of therapy.

Figure 4 is a sectional view of the cover 106, illustrating additional details that may be associated with some embodiments. If the cover 106 is to be removed from the attachment surface 105, the barrier layer 128 may be removed from the film layer 126. Removal of the barrier layer 128 can discontinue the filtering of electromagnetic radiation. Following removal of the barrier layer 128, electromagnetic radiation including both, the violet-blue light 136 and the visible light 134, can propagate through the film layer 126 to the adhesive layer 124. If the adhesive layer 124 contains the photosensitive agents, the photosensitive agents in the adhesive layer 124 may be activated by the violet-blue light 136. If the photosensitive agents are activated by the violet-blue light 136, the adhesive layer 124 becomes further cross-linked. Crosslinking of the adhesive layer 124 may transition the adhesive layer 124 beyond the maximum bond strength of the adhesive layer 124. As a result, the adhesive layer 124 may transition from the second bond strength to the third bond strength. In some embodiments, the third bond strength is less than the second bond strength, facilitating separation and removal of the adhesive layer 124 from the attachment surface 105.

In some embodiments, the barrier layer 128 may have no tinting or no coloring, permitting the barrier layer 128 to be transparent and clear. Photosensitive agents added to the adhesive layer 124 may react to ultraviolet light, i.e., electromagnetic radiation having a wavelength between about 10 nm and about 125 nm. The barrier layer 128 may contain chromophors or other light absorbing groups, such as ClearShield by Milliken or Solarsorb by Croda. The chromophors may absorb electromagnetic radiation having wavelengths up to 390 nm. The chromophors permit the barrier layer 128 to block the ultraviolet light from penetrating through the barrier layer 128 while maintaining the transparency and clarity of the cover 106. In some embodiments, an ultraviolet light source emitting ultraviolet light may be provided with the cover 106. If the barrier layer 128 is removed, the ultraviolet light source may be used to trigger the adhesive layer 124 to transition from the second bond strength to the third bond strength. The transition of the adhesive layer 124 from the second bond strength to the third bond strength can facilitate separation and removal of the adhesive layer 124 from the attachment surface 105.

In some other example embodiments, the barrier layer 128 may include a fluorescing dye, agents, or print. In response to exposure to light having a particular wavelength such as, for example, 450 nm, the barrier layer 128 may fluoresce because of the additives. The fluorescing agents, for example, can provide an indication to a user that the barrier layer 128 has not been removed.

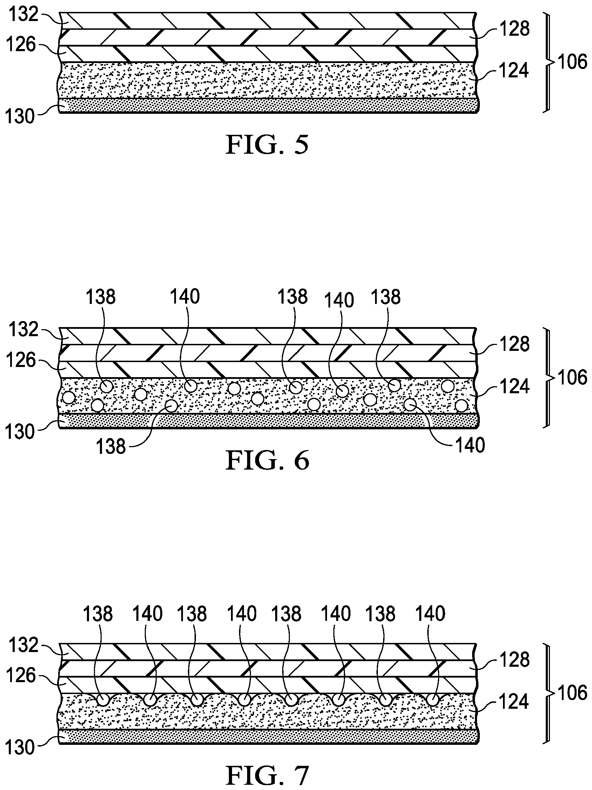

Figure 5 is a sectional view of another embodiment of the cover 106, illustrating additional details that may be associated with some embodiments. The cover 106 may include a release layer 130 and a support layer or carrier layer 132. The release layer 130 may be a siliconized paper layer coupled to a surface of the adhesive layer 124 opposite the film layer 126. The release layer 130 may be configured to protect the adhesive layer 124 prior to use of the cover 106, particularly during shipping and storage of the cover 106. The release layer 130 may be removed prior to use of the cover 106. The carrier layer 132 may be a siliconized paper layer or other a film layer. The carrier layer 132 may be coupled to a surface of the barrier layer 128 opposite the film layer 126. The carrier layer 132 may protect the barrier layer 128 prior to use of the cover 106 and may be removed prior to use of the cover 106.

Figure 6 is a sectional view of another embodiment of the cover 106, illustrating additional details that may be used with some embodiments. The cover 106 may include the adhesive layer 124 coupled to a first side of the film layer 126, and a barrier layer coupled to a second side of the film layer 126. The cover 106 can further include the release layer 130 coupled to a side of the adhesive layer 124 opposite the film layer 126, and the carrier layer 132 coupled to a side of the barrier layer 128 opposite the film layer 126.

In some embodiments, the adhesive layer 124 may include a plurality of microcapsules 138. The microcapsules 138 may be formed from synthetic polymers, such as aminoplasts, or natural polymers, such as gelatin. The microcapsules 138 may be filled with an ink or dye. In some embodiments, the microcapsules 138 may contain a green dye; however, no particular dye color is required. In some embodiments, the microcapsules 138 may be ruptured by the force F, releasing the ink or dye. The ink or dye may stain the adhesive of the adhesive layer 124 to provide a visual indication that the force F has been applied to the adhesive layer 124. In some embodiments, the microcapsules 138 may be configured to rupture if the force F applied to the adhesive layer 124 is about the force F required to crosslink the adhesive layer 124 to transition the adhesive layer 124 form the first bond strength to the second bond strength.

The adhesive layer 124 may also include a plurality of microcapsules 140. The microcapsules 140 may be formed from synthetic polymers, such as aminoplasts, or natural polymers, such as gelatin. The microcapsules 140 may have a photosensitive agent disposed within the material of the microcapsules 140. The microcapsules 140 may be filled with an ink or dye. In some embodiments, the microcapsules 140 may contain a red dye; however, no particular dye color is required. The photosensitive agents disposed in the material forming the microcapsules 140 may be activated by exposure to violet-blue light 136, causing the microcapsules 140 to rupture. In some embodiments, the microcapsules 140 may be configured to rupture after exposure to the violet-blue light 136 for a time period that is about the same as the time period necessary to cause the adhesive layer 124 to transition from the second bond strength to the third bond strength. If the microcapsules 140 rupture in response to exposure to violet-blue light 136, the ink or dye is released, providing an indication to a user that the cover 106 is ready to be removed.

In some embodiments, the adhesive layer 124 may include a fluorescing additive. The fluorescing additive may be configured to fluoresce in the presence of violet-blue light 136, providing an indicator that the barrier layer 128 has been removed from the cover 106. In some embodiments, the fluorescing additive may be similar to Ultra Red ™ provided by Dymax®.

Figure 7 is a sectional view a sectional view of another cover 106, illustrating additional details that may be associated with some embodiments. The cover 106 may include the adhesive layer 124 coupled to a first side of the film layer 126, and a barrier layer coupled to a second side of the film layer 126. The cover 106 can further include the release layer 130 coupled to a side of the adhesive layer 124 opposite the film layer 126, and the carrier layer 132 coupled to a side of the barrier layer 128 opposite the film layer 126. The cover 106 can also include the microcapsules 138 and the microcapsules 140. The microcapsules 138 and the microcapsules 140 may be disposed on a surface of the adhesive layer 124 adjacent to the film layer 126. When ruptured, the color indication provided by the microcapsules 138 and the microcapsules 140 may be visible by a user at a surface of the adhesive layer 124 contacting the film layer 126.

The systems, apparatuses, and methods described herein may provide significant advantages. For example, the covers described herein provide low trauma removal to the patient while also providing a low leak cover for negative-pressure therapy. The covers described can be simple to apply and allow for repositioning before a final seal has been acquired. The covers provided can also seal areas that are difficult to seal. For example, body contours, joints, and wound environments that may wrap around a body can be covered and sealed with the covers described herein. Instillation therapy, negative-pressure therapy, and positive-pressure therapy can all be conducted using the illustrative covers. The covers described can also provide visual cues regarding the state of the drape in sealing and removal readiness.

## Claims

1. A drape for medical applications, the drape comprising:
a film layer (126) having a first side and a second side;
an adhesive layer (124) coupled to the first side of the film layer and comprising microcapsules (138) disposed within the adhesive layer (124), the microcapsules (138) containing tackifiers and being configured to rupture and release the tackifiers in response to a force applied to the drape,
the adhesive layer (124) further comprising one or more photoinitiator or photosensitive agents configured to react to electromagnetic radiation in a visible light spectrum,
the adhesive layer (124) having a first bond strength prior to rupture of the microcapsules (138), a second bond strength after rupture of the microcapsules (138), and a third bond strength following exposure of the photoinitiator or photosensitive agents to electromagnetic radiation in the visible light spectrum; and
a barrier layer (128) releasably coupled to the second side of the film layer (126) and configured to block at least a portion of the electromagnetic radiation in the visible light spectrum in order to prevent premature activation of the photoinitiator or photosensitive agents in the adhesive layer.

2. The drape of claim 1, wherein the adhesive layer (124) comprises an acrylic or polyurethane adhesive.

3. The drape of claim 1, further comprising microcapsules containing adhesive softeners, the microcapsules disposed within the adhesive layer (124) and being configured to rupture and release the adhesive softeners in response to the force.

4. The drape of claim 1, further comprising microcapsules containing a dye, the microcapsules disposed within the adhesive layer (124) and being configured to rupture and release the dye in response to the force.

5. The drape of any of claims 1, 3 or 4, wherein the microcapsules (138) are formed from a material selected from a group consisting of one or more of: synthetic polymers, aminoplasts, natural polymers, and gelatin.

6. The drape of claim 1, wherein the third bond strength is about 50% of the second bond strength; and optionally
a plurality of microcapsules encapsulating a dye are disposed within the adhesive layer (124) and configured to rupture in response to exposure to electromagnetic radiation in the visible light spectrum; or the adhesive layer (124) comprises a fluorescing additive configured to fluoresce in response to exposure to the electromagnetic radiation.

7. The drape of claim 1, wherein the visible light spectrum includes electromagnetic radiation having a wavelength between about 430 nanometers and about 470 nanometers, and the photoinitiator or photosensitive agents are configured to react to electromagnetic radiation having wavelengths between about 430 nanometers and about 470 nanometers.

8. The drape of claim 1, wherein one or more photosensitive agents are selected from the group consisting of: camphorquinone and 5, 7-diiodo-3-butoxy-6-fluoroene.

9. The drape of claim 1, wherein the film layer (126) comprises a polyurethane film layer, optionally further comprising a carboxymethyl cellulose (CMC) disposed within the film layer, optionally wherein the film layer is configured to absorb liquid.

10. The drape of claim 1 or 9, wherein the barrier layer (128) has a yellow tint.

11. The drape of claim 1, further comprising chromophors or electromagnetic radiation absorbing groups disposed within the barrier layer (128).

12. The drape of claim 1, wherein the barrier layer (128) comprises a material selected from the group consisting of one or more of: a highly breathable polymer, polyurethane, polyamide, polyether block amide, ethylene-vinyl acetate, polyvinyl alcohol, hydroxy acrylics, and carboxy acrylics.

13. The drape of claim 1, further comprising a release layer (130) releasably coupled to the adhesive layer (124) opposite the film layer (126).

14. The drape of claim 1 or 13, further comprising a support layer (132) releasably coupled to the barrier layer (128) opposite the film layer (126).

15. A system for providing negative-pressure therapy, the system comprising:
a manifold configured to be positioned over a tissue site;
a drape according to any preceding claim, configured to be positioned over the manifold and sealed to an attachment surface surrounding the tissue site; and
a negative-pressure source (104) configured to be fluidly coupled to the manifold.

## Patentansprüche

1. Abdecktuch für medizinische Anwendungen, wobei das Abdecktuch umfasst:
eine Filmschicht (126) mit einer ersten Seite und einer zweiten Seite;
eine Klebeschicht (124), die mit der ersten Seite der Filmschicht gekoppelt ist und Mikrokapseln (138) umfasst, die innerhalb der Klebeschicht (124) angeordnet sind, wobei die Mikrokapseln (138) Klebrigmacher aufweisen und konfiguriert sind, um als Reaktion auf eine auf das Abdecktuch ausgeübte Kraft zu zerbrechen und die Klebrigmacher freizugeben,
wobei die Klebeschicht (124) weiter einen oder mehrere Photoinitiatoren oder photosensitive Mittel umfasst, die konfiguriert sind, um auf elektro-magnetische Strahlung in einem sichtbaren Lichtspektrum zu reagieren,
wobei die Klebeschicht (124) eine erste Klebefestigkeit vor dem Bruch der Mikrokapseln (138), eine zweite Klebefestigkeit nach dem Bruch der Mikrokapseln (138) und eine dritte Klebefestigkeit nach Einwirkung von elektromagnetischer Strahlung im sichtbaren Lichtspektrum auf den Photoinitiator oder die photosensitiven Mittelaufweist; und
eine Sperrschicht (128), die lösbar mit der zweiten Seite der Filmschicht (126) gekoppelt und konfiguriert ist, um zumindest einen Teil der elektromagnetischen Strahlung in dem sichtbaren Lichtspektrum zu blockieren, um eine vorzeitige Aktivierung des Photoinitiators oder der photosensitiven Mittel in der Klebeschicht zu verhindern.

2. Abdecktuch nach Anspruch 1, wobei die Klebeschicht (124) einen Acryl- oder Polyurethan-Klebstoff umfasst.

3. Abdecktuch nach Anspruch 1, weiter umfassend Mikrokapseln, die Klebstoffweichmacher aufweisen, wobei die Mikrokapseln innerhalb der Klebeschicht (124) angeordnet und konfiguriert sind, um als Reaktion auf die Kraft zu zerbrechen und die Klebstoffweichmacher freizugeben.

4. Abdecktuch nach Anspruch 1, weiter umfassend das Enthalten eines Farbstoffs, wobei die Mikrokapseln innerhalb der Klebeschicht (124) angeordnet und konfiguriert sind, um als Reaktion auf die Kraft zu zerbrechen und den Farbstoff freizugeben.

5. Abdecktuch nach einem der Ansprüche 1, 3 oder 4, wobei die Mikrokapseln (138) aus einem Material gebildet sind, das aus der Gruppe ausgewählt ist, bestehend aus einem oder mehreren von: synthetischen Polymeren, Aminoplasten, natürlichen Polymeren und Gelatine.

6. Abdecktuch nach Anspruch 1, wobei die dritte Klebefestigkeit etwa 50% der zweiten Klebefestigkeit beträgt; und wobei optional
eine Vielzahl von Mikrokapseln, die einen Farbstoff einkapseln, innerhalb der Klebeschicht (124) angeordnet und konfiguriert sind, um als Reaktion auf die Exposition gegenüber elektromagnetischer Strahlung in dem sichtbaren Lichtspektrum zu zerbrechen; oder die Klebeschicht (124) ein fluoreszierendes Additiv umfasst, das konfiguriert ist, um als Reaktion auf die Exposition gegenüber der elektromagnetischen Strahlung zu fluoreszieren.

7. Abdecktuch nach Anspruch 1, wobei das sichtbare Lichtspektrum elektromagnetische Strahlung mit einer Wellenlänge zwischen etwa 430 Nanometern und etwa 470 Nanometern aufweist, und der Photoinitiator oder die photosensitiven Mittel konfiguriert sind, um auf elektromagnetische Strahlung mit Wellenlängen zwischen etwa 430 Nanometern und etwa 470 Nanometern zu reagieren.

8. Abdecktuch nach Anspruch 1, wobei ein oder mehrere photosensitive Mittel aus der Gruppe ausgewählt ist/sind, bestehend aus: Campherchinon und 5, 7-Diiodo-3-Butoxy-6-Fluoroen.

9. Abdecktuch nach Anspruch 1, wobei die Filmschicht (126) eine Polyurethan-Filmschicht umfasst, optional weiter umfassend eine innerhalb der Filmschicht angeordneteCarboxymethylcellulose (CMC), optional wobei die Filmschicht konfiguriert ist, um Flüssigkeit zu absorbieren.

10. Abdecktuch nach Anspruch 1 oder 9, wobei die Sperrschicht (128) eine gelbe Färbung aufweist.

11. Abdecktuch nach Anspruch 1, weiter umfassend Chromophore oder elektromagnetische Strahlung absorbierende Gruppen, die innerhalb der Sperrschicht (128) angeordnet sind.

12. Abdecktuch nach Anspruch 1, wobei die Sperrschicht (128) ein Material umfasst, das aus der Gruppe ausgewählt ist, bestehend aus einem oder mehreren von: einem hochatmungsaktiven Polymer, Polyurethan, Polyamid, Polyetherblockamid, EthylenVinylacetat, Polyvinylalkohol, Hydroxyacrylate und Carboxyacrylate.

13. Abdecktuch nach Anspruch 1, weiter umfassend eine Schutzschicht (130), die gegenüber der Filmschicht (126) lösbar mit der Klebeschicht (124) gekoppelt ist.

14. Abdecktuch nach Anspruch 1 oder 13, weiter umfassend eine Stützschicht (132), die gegenüber der Filmschicht (126) lösbar mit der Sperrschicht (128) gekoppelt ist.

15. System zum Bereitstellen einer Unterdrucktherapie, wobei das System umfasst:
einen Verteiler, der konfiguriert ist, um über einer Gewebestelle positioniert zu werden;
ein Abdecktuch nach einem vorstehenden Anspruch, das konfiguriert ist, um über dem Verteiler positioniert und gegen eine Anbringungsfläche, die die Gewebestelle umgibt, abgedichtet zu werden; und
eine Unterdruckquelle (104), die konfiguriert ist, um mit dem Verteiler strömungsmäßig gekoppelt zu werden.

## Revendications

1. Champ pour des applications médicales, le champ comprenant :
une couche de film (126) ayant un premier côté et un second côté ;
une couche adhésive (124) couplée au premier côté de la couche de film et comprenant des microcapsules (138) disposées à l'intérieur de la couche adhésive (124), les microcapsules (138) contenant des agents poisseux et étant configurées pour se rompre et libérer les agents poisseux en réponse à une force appliquée sur le champ,
la couche adhésive (124) comprenant en outre un ou plusieurs agents photo-initiateurs ou photosensibles configurés pour réagir à un rayonnement électromagnétique dans un spectre de lumière visible,
la couche adhésive (124) ayant une première force de liaison avant rupture des microcapsules (138), une deuxième force de liaison après rupture des microcapsules (138), et une troisième force de liaison après exposition des agents photo-initiateurs ou photosensibles à un rayonnement électromagnétique dans le spectre de lumière visible ; et
une couche barrière (128) couplée de manière amovible au second côté de la couche de film (126) et configurée pour bloquer au moins une partie du rayonnement électromagnétique dans le spectre de lumière visible afin d'empêcher une activation prématurée des agents photo-initiateurs ou photosensibles dans couche adhésive.

2. Champ selon la revendication 1, dans lequel la couche adhésive (124) comprend un adhésif acrylique ou polyuréthane.

3. Champ selon la revendication 1, comprenant en outre des microcapsules contenant des adoucissants adhésifs, les microcapsules étant disposées dans la couche adhésive (124) et étant configurées pour rompre et libérer les adoucissants adhésifs en réponse à la force.

4. Champ selon la revendication 1, comprenant en outre, contenant un colorant, les microcapsules disposées dans la couche adhésive (124) et étant configurées pour rompre et libérer le colorant en réponse à la force.

5. Champ selon l'une quelconque des revendications 1, 3 ou 4, dans lequel les microcapsules (138) sont formées à partir d'un matériau choisi dans un groupe consistant en un ou plusieurs parmi : des polymères synthétiques, des aminoplastes, des polymères naturels, et de la gélatine.

6. Champ selon la revendication 1, dans lequel la troisième force de liaison représente environ 50 % de la deuxième force de liaison ; et facultativement
une pluralité de microcapsules encapsulant un colorant sont disposées dans la couche adhésive (124) et configurées pour se rompre en réponse à une exposition à un rayonnement électromagnétique dans le spectre de lumière visible ; ou la couche adhésive (124) comprend un additif fluorescent configuré pour émettre une fluorescence en réponse à une exposition au rayonnement électromagnétique.

7. Champ selon la revendication 1, dans lequel le spectre de lumière visible inclut un rayonnement électromagnétique ayant une longueur d'onde comprise entre environ 430 nanomètres et environ 470 nanomètres, et les agents photo-initiateurs ou photosensibles sont configurés pour réagir à un rayonnement électromagnétique ayant des longueurs d'onde comprises entre environ 430 nanomètres et environ 470 nanomètres.

8. Champ selon la revendication 1, dans lequel un ou plusieurs agents photosensibles sont choisis dans le groupe consistant en : camphorquinone et 5,7-diiodo-3-butoxy-6-fluoroène.

9. Champ selon la revendication 1, dans lequel la couche de film (126) comprend une couche de film de polyuréthane, comprenant facultativement en outre une carboxyméthylcellulose (CMC) disposée à l'intérieur de la couche de film, facultativement dans lequel la couche de film est configurée pour absorber un liquide.

10. Champ selon la revendication 1 ou 9, dans lequel la couche barrière (128) a une teinte jaune.

11. Champ selon la revendication 1, comprenant en outre des chromophores ou des groupes absorbant un rayonnement électromagnétique disposés à l'intérieur de la couche barrière (128).

12. Champ selon la revendication 1, dans lequel la couche barrière (128) comprend un matériau choisi dans le groupe consistant en un ou plusieurs parmi : un polymère hautement respirant, un polyuréthane, un polyamide, un polyéther bloc amide, un acétate de vinyle-éthylène-, un alcool polyvinylique, des hydroxy-acryliques et des carboxy-acryliques.

13. Champ selon la revendication 1, comprenant en outre une couche anti-adhésive (130) couplée de manière amovible à la couche adhésive (124) opposée à la couche de film (126).

14. Champ selon la revendication 1 ou 13, comprenant en outre une couche de support (132) couplée de manière amovible à la couche barrière (128) opposée à la couche de film (126).

15. Système pour fournir une thérapie par pression négative, le système comprenant :
un collecteur configuré pour être positionné sur un site tissulaire ;
un champ selon l'une quelconque des revendications précédentes, configuré pour être positionné sur le collecteur et scellé à une surface de fixation entourant le site de tissu ; et
une source de pression négative (104) configurée pour être couplée de manière fluidique au collecteur.
